# EUROPEAN PATENT APPLICATION

(11) **EP 4 180 049 A1**
(43) Date of publication of application: **17.05.2023**
(21) Application number: 21208638.3
(22) Date of filing: 16.11.2021
(51) Int. Cl.: A61K 35/17, A61K 35/28, C12N 5/074, C12N 5/0783

(54) **THERAPEUTIC COMPOSITION FOR CANCER TREATMENT**

(71) Applicant: Ostravska univerzita, 70200 Ostrava (CZ)
(72) Inventor: Rodriguez Bago, Julio, 70200 Ostrava (CZ); Hajek, Roman, 63500 Brno (CZ); Charvatova, Sandra, 70030 Ostrava (CZ)
(74) Representative: Harber IP s.r.o.

(57) **Abstract**

The present invention relates to a therapeutic composition comprising a scaffold made of a pharmaceutically acceptable polymer, and natural killer cells and mesenchymal stem cells embedded in the scaffold. The composition in particularly suitable for use in a local postsurgery treatment of cancer or residual cancer.

## Description

### Field of Art

The present invention relates to the field of therapeutic compositions useful in therapy of cancer.

### Background Art

Cancer cell-based therapy is a growing research field that comprises autologous or allogeneic cells to fight cancer and relieve medical conditions. The therapeutic cells' sources employed so far have been variable, going from endothelial progenitor cells (e.g., EP2801583A1) to immune cells (e.g., EP2801583A1). Recent ground-breaking results in treating different hematological malignancies with T-lymphocytes modified with the expression of antigen receptors (US9629877B2) put the cancer cell-based therapy in the spotlight, becoming a pillar in cancer treatment. Still, from the time being, it is a fledgling therapy, and therefore, there are roadblocks to surpass, such as effector toxicity, homing, tumor escape, and universal access.

After surgery to remove cancer, even when a wide-excision is performed, some of the few residual tumor cells left behind can propagate and cause the growth a new tumor. Besides, in many cases, the resected tissue remains unhealed with obvious consequences for the patients' health and quality of life.

The aim of the present invention is to overcome the shortcomings of the currently available technologies.

### Disclosure of Invention

The present invention provides a therapeutic composition comprising a scaffold made of a pharmaceutically acceptable polymer, with natural killer cells and mesenchymal stem cells embedded in the scaffold.

The pharmaceutically acceptable polymer may preferably be protein-based or polysaccharide-based. The polymer should be water-insoluble but biodegradable. Particularly preferably, the scaffold is made of fibrin and/or hyaluronic acid or salts thereof.

The term "scaffold" is generally understood as meaning a three-dimensional structure, engineered to cause desirable cellular interactions and to contribute to the formation of new tissues for medical purposes.

The scaffold provides a support for the embedded cells, and maintains them in the required position, thus focusing their action where it is needed. Fibrin and/or hyaluronic acid or sodium hyaluronate are particularly preferred for the reason of particularly suitable mechanical properties, as well as for the reason of further enhancing the effects of the therapeutic composition. Fibrin is especially preferred.

The scaffold may be prepared prior to application of the NK and MSC cells, or it may be formed *in situ* in a mixture containing the NK and MSC cells. For example, the fibrin scaffold may be formed from fibrinogen by *in situ* cleavage by activated thrombin.

The natural killer cells, known also as large granular lymphocytes, are lymphocytes belonging to the same family as T and B lymphocytes. All three groups develop from a common progenitor. Natural killer cells (NK cells) are classified as group I innate lymphocytes which provide a quick response to a broad range of pathological issues - virally infected cells, cancer cells, even in early stages. NK cells are able to kill cancer cells even without any priming or prior activation. They secrete cytokines such as IFN-gamma and TNF-alpha which further activate additional immune cells and thus enhance immune response.

NK cells can be identified by the presence of CD56 and the absence of CD3.

The mesenchymal stem cells (MSCs) may be classified as multipotent adult stem cells, present typically in umbilical cord, bone marrow and fat tissue. MSCs are known to have an effect on immune cells. According to the prior art knowledge, MSCs produce indoleamine 2,3-dioxygenase (IDO) which inhibits proliferation and cytotoxicity in NK cells, and reduce the expression of NK cell receptors (e.g., Spaggiari GM, Capobianco A, Becchetti S, Mingari MC, Moretta L (February 2006). "Mesenchymal stem cell-natural killer cell interactions: evidence that activated NK cells are capable of killing MSCs, whereas MSCs can inhibit IL-2-induced NK-cell proliferation". Blood. 107 (4): 1484-90). MSCs have a proven regenerative capacity, with the potential of healing damaged tissue after tumor resection.

Within the framework of the present invention, it was observed that MSCs serve as NK cells activators, thus significantly increasing their anticancer properties. An essential asset in both cell types is the possibility to be used in allogeneic approaches, as they do not produce graft versus host disease and have low immunogenicity.

The therapeutic composition according to the invention preferably contains the MSCs to NK cells in the cell number ratio of 1:1 to 1:50, more preferably 1:5 to 1:20, most preferably about 1:10.

The therapeutic composition may be freshly prepared or frozen or lyophilized.

In some embodiments, the NK cells and/or the MSCs may be modified before use (e.g., application to a patient), typically by insertion of genes encoding: apoptosis-inducing ligands, and/or enzymes that convert non-toxic prodrugs into cytotoxic active agents, and/or growing inhibitory proteins and/or immune stimulator cytokines and/or chimeric antigen receptors (CARs). The inserted genes may be under the control of constitutive or conditional promoter.

In particular, the NK cells and/or the MSCs may be modified by insertion of a gene selected from genes encoding TNF-related apoptosis-inducing ligand (TRAIL), Cytosine Deaminase (CD), Herpes simplex virus-thymidine kinase (HSV-TK), IFN- β, IL-15 and CARs capable of recognizing specific tumor antigens such as Erythropoietin-producing Hepatocellular receptor tyrosine kinase class A2 (EphaA2), B-cell maturation antigen (BCMA) and CD19. The inserted genes may be under the control of a constitutive or conditional promoter.

The expression of these genes in the NK cells and/or the MSCs can be transiently or stably integrated into the genome. For the transient expression, non-integrative plasmids can be transfected to the NK cells and/or the MSCs using reagent-based methods such as cationic polymers, DEAE-dextran, calcium phosphate, or instruments based methods such as electroporation. For the stable integration, viral vectors such as lentivirus or retrovirus can be used, as well as non-viral approaches with integrative plasmids with transposon transposase system using reagent-based methods such as cationic polymers, DEAE-dextran, calcium phosphate, or instrument-based methods such as electroporation.

In some embodiments, the NK cells and/or the MSCs may be modified before use (e.g., application to a patient) by loading with anticancer agents, such as anticancer or antitumor drugs, and/or irradiation treatment sensitizers or agents, and/or oncolytic virus. For example, the NK cells and/or the MSCs can be loaded with nanoparticle carriers containing high concentration of insoluble chemotherapeutic agents, such as doxorubicin-containing porous silica nanorattles, by specific antibody-antigen recognitions at the cytomembrane interface.

The therapeutic composition of the present invention is destined for use in the treatment of cancer, more specifically, in a local post-surgery treatment of cancer or residual cancer.

The treatment involves insertion of the therapeutic composition into the place of the resected tissue. The therapeutic composition provides cells which target and kill the residual tumor cells, improve regeneration, and provides a scaffold for new tissue growth.

Particularly preferably, the cancer is selected from multiple myeloma, breast cancer, and osteosarcoma.

The present invention further provides a method for cancer treatment which comprises the steps of resecting tumor, applying the therapeutic composition in the place of the resected tumor, and maintaining the therapeutic composition in the place of the resected tumor until it biodegrades.

The following examples show the preparation as well as the therapeutic effectiveness of the therapeutic composition of the present invention in various types of cancer (represented by multiple myeloma, breast cancer, and osteosarcoma). The examples should not be construed as limiting the scope of the invention.

### Brief Description of Drawings

Figure 1. Anti-tumor efficacy assay, Example 2. Summary graph showing cell viability of tumor cells RPMI 8226 after co-culture with the different composites for 7 days. Data are presented as means ± SD from three technical replicates. *P<0.05 by student's t-test. The data shown is a representative experiment from three independent experiments.
Figure 2. Anti-tumor efficacy assay, Example 2. Summary graph showing cell viability of tumor cells MDA-MB-231 and U2OS after co-culture with the different composites for 7 days. Data are presented as means ± SD from three technical replicates. *P<0.05 by Student's t-test. The data shown is a representative experiment from three independent experiments.
Figure 3. Antitumor efficacy assay, Example 3. Summary graph showing cell viability of tumor cells after co-culture with the composites based on fibrin or hyaluronic acid for 7 days. Data are presented as means ± SD from four technical replicates.
Figure 4. IFN-γ expression in co-cultures of cancer cells with NK cells and with or without MSC, Example 4. Graph showing quantity of IFN-γ in conditioned medium collected after 24 hours of co-culture (n=4).

### Examples of carrying out the Invention

### Example 1: Preparation of the therapeutic composition

NK cells were isolated from peripheral blood buffy coats of healthy donors. First, peripheral blood mononuclear cells were isolated by density gradient centrifugation (Ficoll Paque PLUS, density 1.077 g/mL, GE Healthcare, Chicago, IL, USA). Then, the resulting cells were negatively enriched with NK cells using the NK Cell Isolation Kit (Miltenyi, Bergisch Gladbach, Germany) and the AutoMACS Pro (Magnetic-Activated Cell Sorting, Miltenyi, Bergisch Gladbach, Germany, version 2.3.0.2), following the manufacturer's protocol. The NK cells were cultured in non-adherent flasks with complete NK MACS medium (Miltenyi, Bergisch Gladbach, Germany) supplemented with 5% of human AB serum (Sigma-Aldrich, Munich, Germany), 500 U/mL of interleukin-2 (IL-2; Peprotech, Cranbury, NJ, USA), 100 ng/mL of interleukin-15 (IL-15; Peprotech, Cranbury, NJ, USA), and 0.5% of penicillin-streptomycin (Sigma-Aldrich, Munich, Germany).

To evaluate the NK cells purity, we performed flow cytometry analysis (BD FACSAria^{™} III Cell Sorter (BD Biosciences-US, San Jose, CA, USA, version 2.3.0.2)) of expanded NK cells with the following panel of antibodies: CD56-APC-Cy7-Vio770 (Miltenyi, Bergisch Gladbach, Germany), CD16-APC (Miltenyi, Bergisch Gladbach, Germany), CD3-VioBlue (Miltenyi, Bergisch Gladbach, Germany), TCRg/d-VioBlue (Miltenyi, Bergisch Gladbach, Germany), CD14-VioBlue (Miltenyi, Bergisch Gladbach, Germany), and CD19-VioBlue (Miltenyi, Bergisch Gladbach, Germany).

The resulting analysis confirmed the purity of NK cells derived from peripheral blood characterized by the positive expression of the markers CD56 and CD16, and the negative expression of the markers CD3, TCR, CD14 and CD19.

The MSC derived from the cord blood were obtained from Sigma and expanded with complete DMEM media (Sigma-Aldrich).

Solution A was prepared by combining 86 µl of human AB serum (Sigma-Aldrich), 4 µl of thrombin (250 IU/ml; TISEEL, Baxter), 10 µl of dimethyl sulfoxide for cell culture (DMSO; Panreac AppliChem), 1x10⁶ NK cells and 1x10⁵ MSCs.

Solution B is the sealer protein solution (fibrinogen) (20 mg/ml) (TISEEL, Baxter).

Solutions A and B may be frozen and thawed as needed.

Solutions A and B are applied over the tumor in a ratio of 20 µl of compound B for 100 µl of compound A. Rapid fibrin scaffold is formed when activated thrombin in solution A cleaves the fibrinogen present in solution B.

The product prepared according to Example 1 is called "FINM" in the following examples.

### Example 2: Antitumor efficacy of NKs with MSCs

To assess the anti-tumor efficacy of the therapeutic composition prepared as described in Example 1 and the synergistic effect in the combination of NK cells with MSC, an *in vitro* experiment was performed where the tumor cell line RPMI 8226 (multiple myeloma) viability was monitored by kinetic optical imaging in the following co-cultures: a) NK cells + MSCs + Fibrin (prepared as described in Example 1, FINM), b) NK cells + Fibrin (prepared as described in Example 1, but without the MSCs), c) Fibrin (control, scaffold only).

Each cytotoxic assay was performed in triplicates in flat white 96-well plates with an optical bottom (Thermo Scientific). 2x10⁴ target tumor cells labeled with the expression of luciferase were seeded in each well. On top of them, 20 ul of thawed FINM (consisting of 100 µl aliquots with 86 µl of human AB serum (Sigma-Aldrich), 4 µl of thrombin (250 IU/ml; TISEEL, Baxter), 10 µl of dimethyl sulfoxide for cell culture (DMSO; Panreac AppliChem) and 1x10⁶ NK cells alone or together with 1x10⁵ MSCs) were mixed with 20 ul of sealer protein solution (20 mg/ml) (TISEEL, Baxter). The culture media in all cases was 150 µl of complete RPMI 1640 medium (BioWest). The tumor cell viability in each case was evaluated after 7 days of co-culture by measuring bioluminescence after the addition of D-luciferin potassium salt (Goldbio) to the wells to a final concentration 0.5 mg/ml. Bioluminescence measuring was performed using Infinite^{®} F Plex (Tecan Trading AG) controlled by software i-control^{™} (Tecan).

To stably integrate the expression of firefly luciferase in the tumor cells, we infected them with lentivirus that carries the lentiviral construct with the constitutive expression of the red fluorescence mCherry, and the bioluminescence firefly luciferase reporters. The construct was generated by amplifying the cDNA encoding both reporters from Addgene Plasmid #44965 with specific sequences at the end of the insert that permitted the restriction of the gene with endonucleases (Thermo Fisher Scientific). The resulting product was ligated to the lentivirus backbone (#12262, Addgene, Watertown, MA, USA), previously cut with endonucleases (Thermo Fisher Scientific). The lentivirus construct was packaged as a lentivirus vector in human embryonic kidney 293FT cells as described previously (Motais B, Charvátová S, Walek Z, Hrdinka M, Smolarczyk R, Cichoń T, Czapla J, Giebel S, Šimíček M, Jelinek T, Ševčíková T, Sobotka J, Kořístek Z, Hajek R, Bagó JR. Selection, Expansion, and Unique Pretreatment of Allogeneic Human Natural Killer Cells with Anti-CD38 Monoclonal Antibody for Efficient Multiple Myeloma Treatment. Cells 2021, 10(5), 967). The tumor cells were infected with the lentivirus vector at a varying multiplicity of infection in culture media containing 8 µg/mL of Polybrene (Sigma).

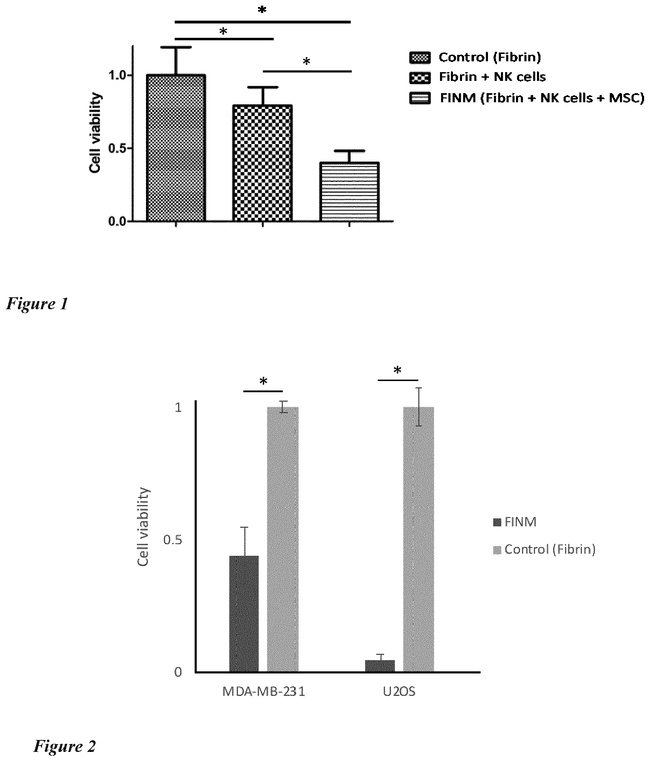

Analysis performed seven days later revealed significant reduction of tumor cells by **50%** in co-culture with the product prepared as described in Example 1 (NK cells + MSCs + Fibrin, FINM) and **25%** in co-culture with NK cells + Fibrin, compared to control with only Fibrin (Figure 1).

The anti-tumor efficacy was also tested *in vitro* against the tumor cell lines MDA-MB-231 (triple-negative breast cancer) and U2OS (osteosarcoma), using the same protocol, and using only Fibrin as control. As shown in Figure 2, seven days after co-culture, a significant reduction in tumor cell viability was observed in both tumor cell lines with FINM, compared with control without FINM. The data indicates the anti-tumor capacity of FINM towards different types of cancer cells.

### Example 3: Antitumor efficacy using various scaffolds

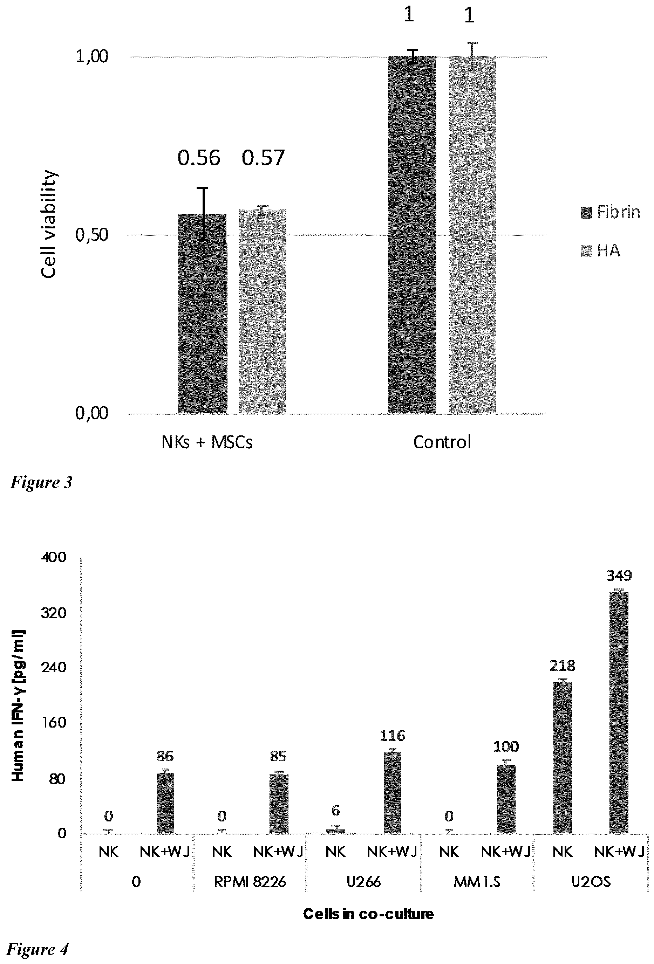

Next, we tested if the synergistic antitumor effect was also observed by combining the NK cells and MSC with other scaffolds such as the FDA-approved Hyaluronic acid (HA). To this end, the tumor viability of cocultures of RPMI 8226 with FINM and with NK cells + MSCs + HA were compared (Figure 3).

The experiment was performed in quadruplicates in flat white 96-well plates with an optical bottom (Thermo Scientific). In each well, 2x10⁴ target tumor cells labeled with luciferase expression were seeded and then covered with FINM by mixing 20 µl of solution A with 4 µl of solution B (compounds composition described in Example 1) or with hyaluronic acid composite prepared as follows: 20 µl of HyStem (Sigma HyStem Cell Culture Scaffold Kit) containing 2x10⁵ NK cells + 2x10⁴ MSCs mixed with 4 µl of Extralink 1 (Sigma HyStem Cell Culture Scaffold Kit).

The culture media in all cases was 150 µl of complete RPMI 1640 medium (BioWest). The viability of cancer cells was evaluated after 7 days of co-culture by measuring bioluminescence induced by adding D-luciferin potassium salt (Goldbio) to the wells to a final concentration of 0.5 mg/ml. Bioluminescence measuring was performed using Infinite^{®} F Plex (Tecan Trading AG) controlled by software i-control^{™} (Tecan).

The data obtained (see Figure 3) indicate that while the scaffolds as such do not have any effect, the scaffolds with embedded NK cells and MSCs show an antitumor effect. The effect is slightly enhanced for fibrin over HA.

### Example 4: Production of IFN-γ by NK cells in the presence of MSCs

In this example, MSCs + NK cells, and as a control NK cells alone, were co-cultured with different tumor cell lines (RPMI 8226, U266, MM1.S, and U2OS) and the expression of INF-γ in each case was quantified.

Conditioned medium from 24-hour-co-cultures of cancer cells (1x10⁴/well), NK cells (2x10⁵/well) with and without MSCs (2x10⁴/well) was collected from each 96-well plate (100 µl/well). IFN-γ from the conditioned media was quantified with the IFN-γ ELISA Kit (Invitrogen) following the manufacture instructions. Absorbance was measured on the microplate reader Infinite F Plex (Tecan) at 450 nm. IFN-γ concentrations were calculated based on the kit's standard curve measured alongside the samples.

As is shown in **Figure 4****,** there is a significant increase in the expression of INF-γ in all co-cultures of NK cells + MSCs, compared to co-cultures with only NK cells. Together, this finding shows that the synergistic therapeutic effect in FINM is partially due to the increase of expression of INF-γ. The remaining part of the synergistic effect was not yet explained by the inventors.

## Claims

1. A therapeutic composition comprising a scaffold made of a pharmaceutically acceptable polymer, and natural killer cells and mesenchymal stem cells embedded in the scaffold.

2. The therapeutic composition according to claim 1, wherein the pharmaceutically acceptable polymer is protein-based or polysaccharide-based, and the pharmaceutically acceptable polymer is water-insoluble and biodegradable.

3. The therapeutic composition according to claim 1, wherein the pharmaceutically acceptable polymer is fibrin and/or hyaluronic acid or salts thereof.

4. The therapeutic composition according to any one of the preceding claims, which contains the mesenchymal stem cells and the natural killer cells in the cell number ratio mesenchymal stem cells to natural killer cells of 1:1 to 1:50, more preferably 1:5 to 1:20, most preferably about 1:10.

5. The therapeutic composition according to any one of the preceding claims, which is frozen or lyophilized.

6. The therapeutic composition according to any one of the preceding claims, wherein the natural killer cells and/or mesenchymal stem cells are modified by insertion of at least one gene encoding an apoptosis-inducing ligand, and/or an enzyme for converting non-toxic prodrugs into cytotoxic products, and/or an growing inhibitory protein, and/or an immune stimulator cytokine, and/or CARs.

7. The therapeutic composition according to any one of claims 1 to 5, wherein the natural killer cells and/or mesenchymal stem cells are modified by insertion of at least one gene selected from genes encoding TRAIL, Cytosine Deaminase (CD), Herpes simplex virus-thymidine kinase (HSV-TK), IFN-β and IL-15 and CARs capable of recognize specific tumor antigens such as Erythropoietin-producing Hepatocellular receptor tyrosine kinase class A2 (EphaA2), B-cell maturation antigen (BCMA) and CD19.

8. The therapeutic composition according to any one of claims 1 to 5, wherein the natural killer cells and/or mesenchymal stem cells are loaded with at least one anticancer agent.

9. The therapeutic composition according to claim 8, wherein the anticancer agent is selected from anticancer drugs, antitumor drugs, irradiation treatment sensitizers, irradiation treatment agents and oncolytic virus.

10. The therapeutic composition according to claim 8, wherein the anticancer agent is a nanoparticle carrier containing at least one insoluble chemotherapeutic agent.

11. The therapeutic composition according to any one of the preceding claims for use in the treatment of cancer.

12. The therapeutic composition according to any one of claims 1 to 10 for use in a local post-surgery treatment of cancer or residual cancer.

13. The therapeutic composition for use according to claim 11 or 12, wherein the cancer is selected from multiple myeloma, breast cancer, and osteosarcoma.
